# FASCICULE DE BREVET EUROPEEN

(11) **EP 0 756 860 B1**
(45) Date de publication et mention de la délivrance du brevet: **06.08.1997**
(21) Numéro de dépôt: 96401292.6
(22) Date de dépôt: 13.06.1996
(51) Int. Cl.: A61K 7/06, A61K 7/50

(54) **Compositions cosmétiques détergentes à usage capillaire et utilisation de ces dernières**
Kosmetisches Haarwaschmittel und dessen Verwendung
Hair cleaning cosmetic composition and its use

(30) Priorité: 07.07.1995 FR 9508271
(43) Date de publication de la demande: 05.02.1997
(73) Titulaire: L'OREAL, 75008 Paris (FR)
(72) Inventeur: Dubief, Claude, 78150 Le Chesnay (FR); Cauwet-Martin, Danièle, 75011 Paris (FR)
(74) Mandataire: Miszputen, Laurent

(56) Documents cités:
- EP-A- 0 219 830
- EP-A- 0 392 320
- EP-A- 0 531 650
- EP-A- 0 582 152
- WO-A-93/23009
- WO-A-94/06409

## Description

La présente invention concerne de nouvelles compositions cosmétiques à propriétés améliorées destinées simultanément au nettoyage, au conditionnement et au coiffage des cheveux, et comprenant, dans un support cosmétiquement acceptable, une base lavante constituée de tensio-actifs à pouvoir détergent dans laquelle sont également présents des agents conditionneurs de type polymères cationiques en association avec des dérivés siliconés particuliers. L'invention concerne aussi l'utilisation desdites compositions dans l'application cosmétique susmentionnée.

Pour le nettoyage et/ou le lavage des cheveux, l'utilisation de compositions capillaires détergentes (ou shampooings) à base essentiellement d'agents tensio-actifs classiques de type notamment anioniques, non-ioniques et/ou amphothères, mais plus particulièrement de type anioniques, est courante. Ces compositions sont appliquées sur cheveux mouillés et la mousse générée par massage ou friction avec les mains permet, après rinçage à l'eau, l'élimination des diverses salissures initialement présentes sur les cheveux.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entrainer à la longue sur la fibre capillaire des dommages plus ou moins marqués liés en particulier à l'élimination progressive des lipides ou protéïnes contenues dans ou à la surface de cette dernière.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abimés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destinés principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, qui confèrent en effet aux cheveux lavés, secs ou mouillés, une facilité de démélage, une douceur et un lissage nettement accrus par rapport à ce qui peut être obtenu avec les compositions nettoyantes correspondantes qui en sont exemptes.

Par ailleurs, depuis quelque temps, on cherche à obtenir des shampooings conditionneurs qui soient capables d'apporter aux cheveux lavés non seulement les propriétés cosmétiques mentionnées ci-avant mais aussi, à un degré plus ou moins poussé, des propriétés de coiffage, de volume, de mise en forme et de tenue. Ces derniers shampooings lavants à propriétés cosmétiques générales améliorées sont souvent dénommés, par simplicité, "shampooings à effets coiffants", expression qui sera d'ailleurs reprise dans la suite de l'exposé.

Toutefois, et malgré les progrès réalisés récemment dans le domaine des shampooings à effets coiffants à base de polymères cationiques, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoqués ci-avant, de meilleures performances.

La présente invention vise à la satisfaction d'un tel besoin.

Ainsi, à la suite d'importantes recherches menées sur la question, il a maintenant été trouvé par la Demanderesse, de façon totalement inattendue et surprenante, qu'en introduisant des dérivés siliconés particuliers et convenablement sélectionnés, tels que définis ci-après, dans des compositions capillaires détergentes contenant des polymères cationiques à titre d'agents conditionneurs, il est possible d'améliorer de manière substantielle et significative les propriétés cosmétiques attachées à ces dernières, et ceci tout en conservant leur bon pouvoir lavant intrinsèque.

Sans vouloir limiter la présente invention à une quelconque théorie, il semblerait exister entre les polymères cationiques, les dérivés siliconés conformes à l'invention et les cheveux, des interactions et/ou des affinités particulières qui favorisent un dépôt régulier, important et durable desdits dérivés siliconés à la surface desdits cheveux, ce dépôt qualitatif et quantitatif étant probablement l'une des causes de l'amélioration observée au niveau des propriétés cosmétiques finales, en particulier la facilité de coiffage, le maintien, la nervosité et le volume des cheveux traités. En tout état de cause, les propriétés cosmétiques attachées aux bases lavantes contenant l'association d'agents [polymère cationique/dérivé siliconé spécifique] conforme à l'invention sont nettement supérieures à celles qui peuvent être obtenues en ne mettant en oeuvre qu'un seul de ces agents à concentration globale équivalente.

Toutes ces découverte sont à la base la présente invention.

Ainsi, selon la présente invention, il est maintenant proposé de nouvelles compositions capillaires détergentes, du type comprenant, dans un milieu cosmétiquement acceptable, une base lavante et au moins un polymère cationique, et qui sont essentiellement caractérisées par le fait qu'elles contiennent en outre au moins un dérivé siliconé hydrosoluble ou hydrodispersible comprenant une chaine principale siliconée sur laquelle est greffé au moins un groupement hydrocarboné à caractère anionique.

L'invention a également pour objet l'utilisation en cosmétique des compositions ci-dessus pour le nettoyage, le conditionnement et le coiffage des cheveux. Mais d'autres caractéristiques, aspects et avantages de l'invention apparaitront encore plus clairement à la lecture de la description qui va suivre ainsi que des exemples concrets, mais nullement limitatifs, destinés à l'illustrer.

Comme indiqué précédemment, les constituants essentiels rentrant dans la composition des produits capillaires de l'invention sont (i) le ou les tensioactifs à pouvoir détergent destinés à former la base lavante, (ii) le ou les agents conditionneurs de type polymères cationiques et (iii) le ou les dérivés siliconés hydrosolubles ou hydrodispersibles spécifiques.

### A- BASE LAVANTE :

Les compositions conformes à l'invention comprennent nécessairement une base lavante, généralement aqueuse.

Le ou les tensioactifs formant la base lavante peuvent être indifféremment choisis, seuls ou en mélanges, au sein des tensioactifs anioniques, amphotères, non ioniques, zwittérioniques et cationiques.

La quantité minimale de base lavante est celle juste suffisante pour conférer à la composition finale un pouvoir moussant et/ou détergent satisfaisant, et des quantités trop importantes de base lavante n'apportent pas vraiment d'avantages supplémentaires.

Ainsi, selon l'invention, la base lavante peut représenter de 4 % à 30 % en poids, de préférence de 10 % à 25 % en poids, et encore plus préférentiellement de 12 % à 20 % en poids, du poids total de la composition finale.

Les tensioactifs convenant à la mise en oeuvre de la présente invention sont notamment les suivants :

### (i) Tensioactif(s) anionique(s) :

Leur nature ne revet pas, dans le cadre de la présente invention, de caractère véritablement critique.

Ainsi, a titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides éthers carboxyliques polyoxyalkylénés et leurs sels, en particulier ceux comportant de 2 à 50 groupements oxyde d'éthylène, et leurs mélanges. Les tensioactifs anioniques du type acides ou sels d'éthers carboxyliques polyoxyalkylénés sont en particulier ceux qui répondent à la formule (1) suivante :

R₁(̵OC₂H₄)̵ₙOCH₂COOA (1)

dans laquelle :
R₁ désigne un groupement alkyle ou alkylaryle, et n est un nombre entier ou décimal (valeur moyenne) pouvant varier de 2 à 24 et de préférence de 3 à 10, le radical alkyle ayant entre 6 et 20 atomes de carbone environ, et aryle désignant de préférence phényle,

A désigne H, ammonium, Na, K, Li, Mg ou un reste monoéthanolamine ou triéthanolamine. On peut également utiliser des mélanges de composés de formule (1) en particulier des mélanges dans lesquels les groupements R₁ sont différents.

Des composés de formule (1) sont vendus par exemple par la Société CHEM Y sous les dénominations AKYPOS (NP40, NP70, OP40, OP80, RLM25, RLM38, RLMQ 38 NV, RLM 45, RLM 45 NV, RLM 100, RLM 100 NV, RO 20, RO 90, RCS 60, RS 60, RS 100, RO 50) ou par la Société SANDOZ sous les dénominations SANDOPAN (DTC Acid, DTC).

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀-C₁₄) amines ou les oxydes de N-acylaminopropylmorpholine. On notera que les alkylpolyglycosides constituent des tensio-actifs non-ioniques rentrant particulièrement bien dans le cadre de la présente invention.

### (iii) Tensioactif(s) amphotère(s) ou zwittérionique(s) :

Les agents tensioactifs amphotères ou zwitterioniques, dont la nature ne revet pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 18 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) betaïnes ou les alkyl (C₈-C₂₀) amidoalkyl (C₁-C₆) sulfobétaïnes.

Parmi les dérivés d'amines, on peut citer les produits vendus sous la dénomination MIRANOL, tels que décrits dans les brevets US-2 528 378 et US-2 781 354 et classés dans le dictionnaire CTFA, 3ème édition, 1982, sous les dénominations Amphocarboxyglycinates et Amphocarboxypropionates de structures respectives :

R₂-CONHCH₂CH₂-N(R₃)(R₄)(CH₂COO-) (2)

dans laquelle : R₂ désigne un radical alkyle d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle;
et 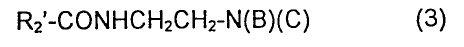 dans laquelle :
B représente -CH₂CH₂OX', C représente -(CH₂)_{z}-Y', avec z = 1 ou 2,
X' désigne le groupement -CH₂CH₂-COOH ou un atome d'hydrogène
Y' désigne -COOH ou le radical -CH₂ - CHOH - SO₃H
R₂ 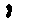 désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

A titre d'exemple on peut citer le cocoamphocarboxyglycinate vendu sous la dénomination commerciale MIRANOL C2M concentré par la Société MIRANOL.

### (iv) Tensioactifs cationiques :

Parmi les tensioactifs cationiques on peut citer en particulier (liste non limitative) : les sels d'amines grasses primaires, secondaires ou tertiaires, éventuellement polyoxyalkylénées; les sels d'ammonium quaternaire tels que les chlorures ou les bromures de tétraalkylammonium, d'alkylamidoalkyltrialkylammonium, de trialkylbenzylammonium, de trialkylhydroxyalkylammonium ou d'alkylpyridinium; les dérivés d'imidazoline ; ou les oxydes d'amines à caractère cationique.
On notera que les tensioactifs cationiques, dont l'utilisation n'est pas exclue, ne constituent pas des tensioactifs préférés pour la mise en oeuvre de la présente invention.

### B- POLYMERE(S) CATIONIQUE(S) :

Les compositions conformes à l'invention comprennent en outre nécessairement un polymère cationique.

Les agents conditionneurs de type polymères cationiques utilisables conformément à la présente invention peuvent être choisis parmi tous ceux déja connus en soi comme améliorant les propriétés cosmétiques des cheveux traités par des compositions détergentes, à savoir notamment ceux décrits dans la demande de brevet EP-A- 0 337 354 et dans les demandes de brevets français FR-A- 2 270 846, 2 383 660, 2 598 611, 2 470 596 et 2 519 863.

De manière encore plus générale, au sens de la présente invention, l'expression "polymère cationique" désigne tout polymère contenant des groupements cationiques ou des groupements ionisables en groupements cationiques.

Les polymères cationiques préférés sont choisis parmi ceux qui contiennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse moléculaire comprise entre 500 et 5.10³ environ, et de préférence comprise entre 10³ et 3.10⁶ environ.

Parmi les polymères cationiques, on peut citer plus particulièrement les protéines (ou hydrolysats de protéines) quaternisées, les polysiloxanes quaternisés et les polymères du type polyamine, polyaminoamide et polyammonium quaternaire. Ce sont des produits connus.

Les protéines ou hydrolysats de protéines quaternisés sont en particulier des polypeptides modifiés chimiquement portant en bout de chaîne, ou greffés sur celle-ci, des groupements ammonium quaternaire. Leur masse moléculaire peut varier par exemple de 1 500 à 10 000, et en particulier de 2 000 à 5 000 environ. Parmi ces composés, on peut citer notamment :
- les hydrolysats de collagène portant des groupements triéthylammonium tels que les produits vendus sous la dénomination "Quat-Pro E" par la Société MAYBROOK et dénommés dans le dictionnaire CTFA "Triéthonium Hydrolyzed Collagen Ethosulfate" ;
- les hydrolysats de collagène portant des groupements chlorure de triméthylammonium et de triméthylstéarylammonium, vendus sous la dénomination de "Quat-Pro S" par la Société Maybroook et dénommnés dans le dictionnaire CTFA "Steartrimonium Hydrolyzed Collagen" ;
- les hydrolysats de protéines animales portant des groupements triméthylbenzylammonium tels que les produits vendus sous la dénomination "Crotein BTA" par la Société CRODA et dénommés dans le dictionnaire CTFA "Benzyltrimonium hydrolyzed animal protein";
- les hydrolysats de protéines portant sur la chaîne polypeptidique des groupements ammonium quaternaire comportant au moins un radical alkyle ayant de 1 à 18 atomes de carbone.

Parmi ces hydrolysats de protéines, on peut citer entre autres :
- le "Croquat L" dont les groupements ammonium quaternaires comportent un groupement alkyle en C₁₂ ;
- le "Croquat M" dont les groupements ammonium quaternairees comportent des groupements alkyle en C₁₀-C₁₈ ;
- le "Croquat S" dont les groupements ammonium quaternaires comportent un groupement alkyle en C₁₈ ;
- le "Crotein Q" dont les groupements ammonium quaternaires comportent au moins un groupe alkyle ayant de 1 à 18 atomes de carbone.

Ces différents produits sont vendus par la Société Croda.

D'autre protéines ou hydrolysats quaternisés sont par exemple ceux répondant à la formule : 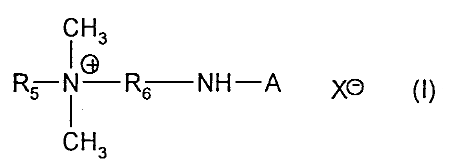 dans laquelle X⁻ est un anion d'un acide organique ou minéral, A désigne un reste de protéine dérivé d'hydrolysats de protéine de collagène, R₅ désigne un groupement lipophile comportant jusqu'à 30 atomes de carbone, R₆ représente un groupement alkylène ayant 1 à 6 atomes de carbone. On peut citer par exemple les produits vendus par la Société Inolex, sous la dénomination "Lexein QX 3000", appelé dans le dictionnaire CTFA "Cocotrimonium Collagent Hydrolysate".

On peut encore citer les protéines végétales quaternisées telles que les protéines de blé, de maïs ou de soja : comme protéines de blé quaternisées on peut citer celles commercialisées par la Société Croda sous les dénominations "Hydrotriticum WQ ou QM", appelées dans le dictionnaire CTFA "Cocodimonium Hydrolysed wheat protein", "Hydrotriticum QL" appelée dans le dictionnaire CTFA "Laurdimonium hydrolysed wheat protein", ou encore "Hydrotriticum QS", appelée dans le dictionnaire CTFA "Steardimonium hydrolysed wheat protein".

Une autre famille de polymères cationiques est celle des polymères cationiques siliconés. Parmi ces polymères, ont peut citer :
(a) les polysiloxanes quaternisés dénommés dans le dictionnaire CTFA "amodiméthicone" et répondant à la formule : 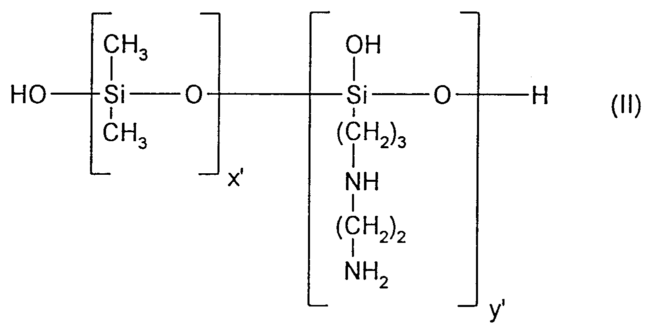 dans laquelle x' et y' sont des nombres entiers dépendant du poids moléculaire, généralement tels que ledit poids moléculaire est compris entre 5 000 et 10 000 environ ;
(b) les polymères cationiques siliconés répondant à la formule :

   R'ₐG₃₋ₐ-Si(OSiG₂)ₙ-(OSiG_{b}R'_{2-b})ₘ-O-SiG₃₋ₐR'ₐ (III)

   dans laquelle :
   G est un atome d'hydrogène, ou un groupement phényle, OH, ou alkyle en C₁-C₈, par exemple méthyle,
   a désigne le nombre 0 ou un nombre entier de 1 à 3, en particulier 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000 et notamment de 1 à 10 ;
   R' est un radical monovalent de formule -C_{q}H_{2q}L dans laquelle q est un nombre de 2 à 8 et L est un groupement aminé éventuellement quaternisé choisi parmi les groupements :
      -NR''-CH₂-CH₂-N'(R'')₂
      -N(R'')2
      -N^{⊕}(R'')₃A⁻
      -N^{⊕}(R'')₃A⁻
      -N^{⊕}(R'')³A⁻
      -N(R'')-CH₂-CH₂-N^{⊕}R''H₂A⁻,
      dans lesquels R'' peut désigner hydrogène, phényle, benzyle, ou un radical hydrocarboné saturé monovalent, par exemple un radical alkyle ayant de 1 à 20 atomes de carbone et A⁻ représente un ion halogénure tel que par exemple fluorure, chlorure, bromure ou iodure.

   Un produit correspondant à cette définition est le polymère dénommé "triméthylsilylamodiméthicone", répondant à la formule : 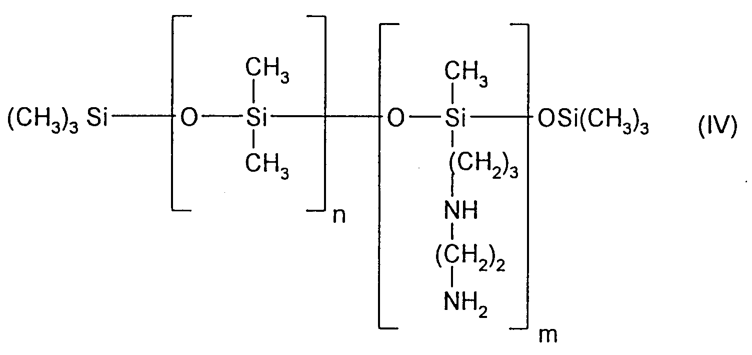 dans laquelle n et m ont les significations données ci-dessus (cf formule III). De tels polymères sont décrits par exemple dans la demande de brevet EP-A-95238.
(c) les polymères cationiques siliconés répondant à la formule : 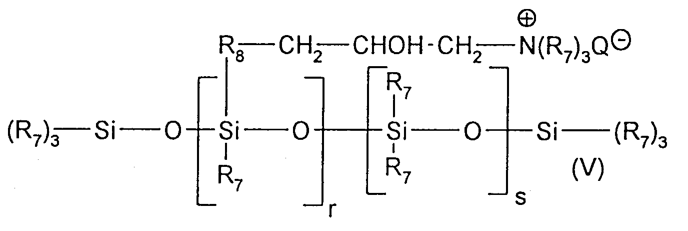 dans laquelle :
   R₇ représente un radical hydrocarboné monovalent ayant de 1 à 18 atomes de carbone, et en particulier un radical alkyle en C₁-C₁₈, ou alcényle en C₂-C₁₈, par exemple méthyle ;
   R₈ représente un radical hydrocarboné divalent, notamment un radical alkylène en C₁-C₁₈ ou un radical alkylèneoxy divalent en C₁-C₁₈, par exemple en C₁-C₈ ;
   Q⁻ est un on halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels polymères sont décrits plus particulièrement dans le brevet US 4 185 087.

Un polymère entrant dans cette classe est le polymère vendu par la Société Union Carbide sous la dénomination "Ucar Silicone ALE 563.

Lorsque ces polymères siliconés sont mis en oeuvre, une forme de réalisation particulièrement intéressante est leur utilisation conjointe avec des agents de surface cationiques et/ou non ioniques. On peut utiliser par exemple le produit vendu sous la dénomination "Emulsion Cationique DC 929" par la Société Dow Corning qui comprend, outre l'amodiméthicone, un agent de surface cationique comprenant un mélange de produits répondant à la formule : 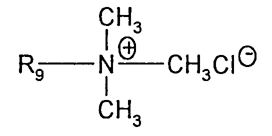 dans lequel R₉ désigne des radicaux alcényle et/ou alcoyle ayant de 14 à 22 atomes de carbone, dérivés des acides gras du suif,
en association avec un agent de surface non ionique de formule :

C₉H₁₉-C₆H₄-(OC₂H₄)₁₀-OH

connu sous la dénomination "Nonoxynol 10".

Un autre produit commercial utilisable selon l'invention est le produit vendu sous la dénomination "Dow Corning Q2 7224" par la Société Dow Corning comportant en association le triméthylsilylamodiméthicone de formule (IV), un agent de surface non ionique de formule : C₈H₁₇-C₆H₄-(OCH₂CH₂)ₙ-OH où n = 40
dénommé encore octoxynol-40,
un autre agent de surface non ionique de formule : C₁₂H₂₅-(OCH₂-CH₂)ₙ-OH où n = 6 encore dénommé isolaureth-6,
et du glycol.

Les polymères du type polyamine, polyamidoamide, polyammonium quaternaire, utilisables conformément à la présente invention, pouvant être notamment mentionnés, sont ceux décrits dans les brevets français n° 2 505 348 ou 2 542 997. Parmi ces polymères, on peut citer :
(1) Les copolymères vinylpyrrolidone-acrylate ou -méthacrylate de dialkylamino-alkyle quaternisés ou non, tels que les produits vendus sous la dénomination "Gafquat" par la Société GAF Corporation, comme par exemple Gafquat 734, 755 ou HS100 ou bien le produit dénommé "Copolymère 937". Ces polymères sont décrits en détail dans les brevets français 2 077 143 et 2 393 573.
(2) Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société Union Carbide Corporation. Ces polymères sont également définis dans le dictionnaire CTFA comme des ammonium quaternaires d'hydroxyéthylcellulose ayant réagi avec un époxyde substitué par un groupement triméthylammonium.
(3) Les dérivés de cellulose cationiques tels que les copolymères de cellulose ou les dérivés de cellulose greffés avec un monomère hydrosoluble d'ammonium quaternaire, et décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylamidopropyl triméthylammonium, de diméthyldiallylammonium.
   Les produits commercialisés répondant à cette définition sont plus particulièrement les produits vendus sous la dénomination "Celquat L 200" et "Celquat H 100" par la Société National Starch.
(4) Les polysaccharides cationiques décrits plus particulièrement dans les brevets US 3 589 578 et 4 031 307 et plus particulièrement le produit commercialisé sous la dénomination "Jaguar C.13 S" vendu par la Société Meyhall.
(5) les polymères constitués de motifs pipérazinyle et de radicaux divalents alkylène ou hydroxyalkylène à chaînes droites ou ramifiées, éventuellement interrompues par des atomes d'oxygène, de soufre, d'azote ou par des cycles aromatiques ou hétérocycliques, ainsi que les produits d'oxydation et/ou de quaternisation de ces polymères. De tels polymères sont notamment décrits dans les brevets français 2.162.025 et 2.280.361 ;
(6) les polyaminoamides solubles dans l'eau préparés en particulier par polycondensation d'un composé acide avec une polyamine ; ces polyaminoamides peuvent être réticulés par une épihalohydrine, un diépoxyde, un dianhydride, un dianhydride non saturé, un dérivé bis-insaturé, une bis-halohydrine, un bis-azétidinium, une bis-haloacyldiamine, un bis-halogénure d'alkyle ou encore par un oligomère résultant de la réaction d'un composé bifonctionnel réactif vis-à-vis d'une bis-halohydrine, d'un bis-azétidinium, d'une bis-haloacyldiamine, d'un bis-halogénure d'alkyle, d'une épilhalohydrine, d'un diépoxyde ou d'un dérivé bis-insaturé ; l'agent réticulant étant utilisé dans des proportions allant de 0,025 à 0,35 mole par groupement amine du polymaoamide ; ces polyaminoamides peuvent être alcoylés ou s'ils comportent une ou plusieurs fonctions amines tertiaires, quaternisées. De tels polymères sont notamment décrits dans les brevets français 2.252.840 et 2.368.508 ;
(7) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-dialcoylaminohydroxydialcoylène triamine dans lesquels le radical alcoyle comporte de 1 à 4 atomes de carbone et désigne de préférence méthyle, éthyle, propyle. De tels polymères sont notamment décrits dans le brevet français 1.583.363.
   Parmi ces dérivés, on peut citer plus particulièrement les polymères acide adipique/diméthylaminohydroxypropyl/diéthyl triamine vendus sous la dénomination "Cartaretine F, F4 ou F8" par la société Sandoz.
(8) les polymères obtenus par réaction d'une polyalkylène polyamine comportant deux groupements amine primaire et au moins un groupement amine secondaire avec un acide dicarboxylique choisi parmi l'acide diglycolique et les acides dicarboxyliques aliphatiques saturés ayant de 3 à 8 atomes de carbone. Le rapport molaire entre le polyalkylène polylamine et l'acide dicarboxylique étant compris entre 0,8 : 1 et 1,4 : 1; le polyaminoamide en résultant étant amené à réagir avec l'épichlorhydrine dans un rapport molaire d' épichlorhydrine par rapport au groupement amine secondaire du polyaminoamide compris entre 0,5 : 1 et 1,8 : 1. De tels polymères sont notamment décrits dans les brevets américains 3.227.615 et 2.961.347.
   Des polymères de ce type sont en particulier commercialisés sous la dénomination "Hercosett 57" par la société Hercules Inc. ou bien sous la dénomination de "PD 170" ou "Delsette 101" par la société Hercules dans le cas du copolymère d'acide adipique/époxypropyl/diéthylènetriamine.
(9) les cyclohomopolymères de méthyl diallyl amine ou de diméthyl diallyl ammonium tels que les homopolymères comportant comme constituant principal de la chaîne des motifs répondant aux formules (VI) ou (VI') : 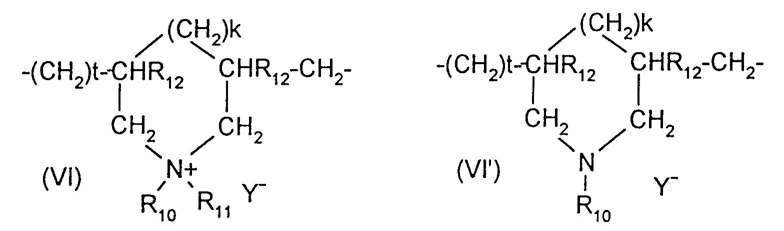 formules dans lesquelles k et t sont égaux à 0 ou 1, la somme k + t étant égale à 1 ; R₁₂ désigne un atome d'hydrogène ou un radical méthyle; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   Parmi les polymères définis ci-dessus, on peut citer plus particulièrement l'homopolymère de chlorure de diméthyldiallylammonium vendu sous la dénomination "Merquat 100" par la société Merck.
(10) le polymère de diammonium quaternaire contenant des motifs récurants répondant à la formule : 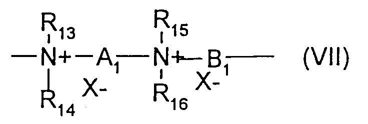 formule (VII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙ-CO-D-OC-(CH₂)ₙ-dans lequel D désigne :
      a) un reste de glycol de formule : -O-Z-O-, où Z désigne un radical hydrocarboné linéaire ou ramifié ou un groupement répondant à l'une des formules suivantes :
         -(CH₂-CH₂-O)ₓ-CH₂-CH₂-
         -[CH₂CH(CH₃)-O]_{y}-CH₂-CH(CH₃)-
         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent -CH₂-CH₂-S-S-CH₂-CH₂- ;
      d) un groupement uréylène de formule : -NH-CO-NH- ;

   De préférence, X⁻ est un anion tel que le chlorure ou le bromure. Ces polymères ont une masse moléculaire généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
(11) les polymères de polyammonium quaternaires constitués de motifs de formule (VIII): 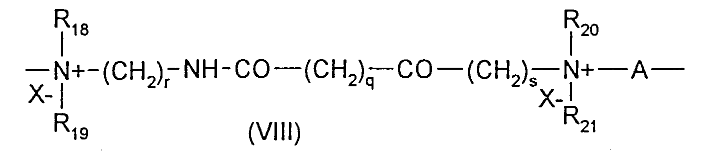 formule dans laquelle :
   R₁₈, R₁₉,
   R₂₀ et R₂₁, identiques ou différents, représentent un atome d'hydrogène ou un radical méthyle, éthyle, propyle, β-hydroxyéthyle, β-hydroxypropyle ou - CH₂CH₂(OCH₂CH₂)ₚOH,
   où p est égal à 0 ou à un nombre entier compris entre 1 et 6, sous réserve que R₁₈, R₁₉, R₂₀ et R₂₁ ne représentent pas simultanément un atome d'hydrogène,
   r et s, identiques ou différents, sont des nombres entiers compris entre 1 et 6,
   q est égal à 0 ou à un nombre entier compris entre 1 et 34,
   X désigne un atome d'halogène,
   A désigne un radical d'un dihalogénure ou représente de préférence -CH₂-CH₂-O-CH₂-CH₂-.

   De tels composés sont notamment décrits dans la demande de brevet EP-A-122 324.
   On peut par exemple citer parmi ceux-ci, les produits "Mirapol A 15", "Mirapol AD1", "Mirapol AZ1" et "Mirapol 175" vendus par la société Miranol.
(12) les homopolymères ou copolymères dérivés des acides acrylique ou méthacrylique et comportant des motifs : 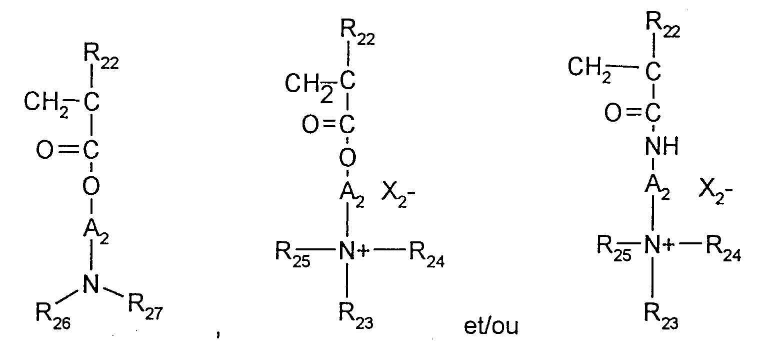 dans lesquels les groupements R₂₂ désignent indépendamment H ou CH₃,
   les groupements A₁ désignent indépendamment un groupe alcoyle linéaire ou ramifié de 1 à 6 atomes de carbone ou un groupe hydroxyalcoyle de 1 à 4 atomes de carbone,
   les groupements R₂₃, R₂₄, R₂₅, identiques ou différents, désignant indépendamment un groupe alcoyle de 1 à 18 atomes de carbone ou un radical benzyle,
   les groupements R₂₆ et R₂₇ représentent unatome d'hydrogène ou un groupement alcoyle de 1 à 6 atomes de carbone,
   X₂⁻ désigne un anion, par exemple méthosulfate ou halogénure, tel que chlorure ou bromure.

   Le ou les comonomères utilisables dans la préparation des copolymères correspondants appartiennent à la famille des acrylamides, méthacrylamides, diacétone acrylamides, acrylamides et méthacrylamides substitués à l'azote par des alcoyle inférieurs, des esters d'alcoyles, des acides acrylique ou méthacrylique, la vinylpyrrolidone ou des esters vinyliques.
(13) Les polymères quaternaires de vinylpyrrolidone et de vinylimidazole tels que par exemple les produits commercialisés sous les dénominations Luviquat FC 905, FC 550 et FC 370 par la société B.A.S.F..
(14) Les polyamines comme le Polyquart H vendu par HENKEL, référencé sous le nom de 〈〈POLYETHYLENEGLYCOL (15) TALLOW POLYAMINE〉〉 dans le dictionnaire CTFA.
(15) Les polymères réticulés de chlorure de méthacryloyloxyéthyltriméthylammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de 〈〈SALCARE SC 92〉〉 par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale. Cette dispersion est commercialisée sous le nom de 〈〈SALCARE SC 95〉〉 par la Société ALLIED COLLOIDS.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Selon l'invention, on peut utiliser plus particulièrement les polymères choisis parmi le Mirapol, le composé de formule (VII) dans laquelle R₁₃, R₁₄, R₁₅ et R₁₆ représentent le radical méthyle, A₁ représente le radical de formule -(CH₂)₃-et B₁ représente le radical de formule -(CH₂)₆- et X⁻ représente l'anion chlorure (nommé ultérieurement Mexomère PO) et le composé de formule (VII) dans laquelle R₁₃ et R₁₄ représentent le radical éthyle, R₁₅ et R₁₆ représentent le radical méthyle, A₁ et B₁ représentent le radical de formule -(CH₂)₃- et X⁻représente l'anion bromure (nommé ultérieurement Mexomère PAK).

Parmi tous les polymères cationiques susceptibles d'être utilisés dans le cadre de la présente invention, on préfère mettre en oeuvre les dérivés d'éther de cellulose quaternaires tels que les produits vendus sous la dénomination «JR 400» par la Société UNION CARBIDE CORPORATION, les cyclopolymères, en particulier les copolymères du chlorure de diméthyldiallylammonium et d'acrylamide ayant un poids moléculaire supérieur à 500 000, vendus sous les dénominations 〈〈MERQUAT 550〉〉 et 〈〈MERQUAT S〉〉 par la Société MERCK, les polysaccharides cationiques et plus particulièrement le polymère vendu sous la dénomination 〈〈JAGUAR C13S〉〉 par la Société MEYHALL, et enfin les polymères cationiques siliconés.

Selon l'invention, le ou les polymères cationiques peuvent représenter de 0,01 % à 10 % en poids, de préférence de 0,05 % à 5 % en poids, et encore plus préférentiellement de 0,1 % à 3 % en poids, du poids total de la composition finale.

### C- DERIVE(S) SILICONE(S) :

Selon une caractéristique essentielle des compositions capillaires détergentes conformes à l'invention, ces dernières contiennent en outre au moins un dérivé siliconé spécifique, hydrosoluble ou hydrodispersible.

Dans ce qui suit, on entend désigner par silicone, en conformité avec l'acception générale, tous polymères ou oligomères organosiliciés à structure linéaire ou cyclique, ramifiée ou réticulée, de poids moléculaire variable, obtenus par polymérisation et/ou polycondensation de silanes convenablement fonctionnalisés, et constitués pour l'essentiel par une répétition de motifs principaux dans lesquels les atomes de silicium sont reliés entre eux par des atomes d'oxygène (liaison siloxane ≡Si-O-Si≡), des radicaux hydrocarbonés éventuellement substitués étant directement liés par l'intermédiaire d'un atome de carbone sur lesdits atomes de silicicium. Les radicaux hydrocarbonés les plus courants sont les radicaux alkyles notamment en C₁-C₁₀ et en particulier méthyle, les radicaux fluoroalkyles, les radicaux aryles et en particulier phényle, et les radicaux alcényles et en particulier vinyle; d'autres types de radicaux susceptibles d'être liés soit directement, soit par l'intermédiaire d'un radical hydrocarboné, à la chaine siloxanique sont notamment l'hydrogène, les halogènes et en particulier le chlore, le brome ou le fluor, les thiols, les radicaux alcoxys, les radicaux polyoxyalkylènes (ou polyéthers) et en particulier polyoxyéthylène et/ou polyoxypropylène, les radicaux hydroxyles ou hydroxyalkyles, les groupements aminés substitués ou non, les groupements amides, les radicaux acyloxys ou acyloxyalkyles, les radicaux hydroxyalcylamino ou aminoalkyles, des groupements ammonium quaternaires, des groupements amphotères ou bétaïniques, des groupements anioniques tels que carboxylates, thioglycolates, sulfosuccinates, thiosulfates, phosphates et sulfates, cette liste n'étant bien entendu nullement limitative (silicones dites "organomodifiées").

Selon la présente invention, le ou les dérivés siliconés qui doivent être utilisés sont ceux qui sont comprennent une chaine principale de silicone (ou polysiloxane (≡Si-O-)ₙ) sur laquelle se trouve greffé, à l'intérieur de ladite chaine ainsi qu'éventuellement à l'une au moins de ses extrémités, au moins un groupement hydrocarboné à caractère anionique. Au sens de la présente invention, on entend par groupement hydrocarboné 〈〈à caractère anionique〉〉, tout groupement hydrocarboné contenant au moins un motif anionique, ou au moins un motif ionisable en motif anionique. Selon une caractétistique essentielle de la présente invention, la nature et/ou la quantité desdits groupements hydrocarbonés à caractère anionique greffés sur la chaine silicone doivent être choisies de manière telle que le dérivé siliconé correspondant soit hydrosoluble ou hydrodispersible, après éventuelle neutralisation des groupements à caractère anionique au moyen d'un agent alcalin. Ces dérivés siliconés particulier peuvent être des produits commerciaux existants, ou encore être obtenus selon tout moyen connu de l'homme de l'art, en particulier par réaction entre (i) une silicone de départ correctement fonctionalisée sur un ou plusieurs de ces atomes de silicium et (ii) un composé anionique lui-même correctement fonctionalisé par une fonction qui est capable de venir réagir avec le ou les groupements fonctionels portés par ladite silicone en formant une liaison covalente ; un exemple classique d'une telle réaction est la réaction d'hydrosylilation entre des groupements ≡Si-H et des groupements vinyliques CH₂=CH-, ou encore la réaction entre des groupements thio-fonctionels -SH avec ces mêmes groupements vinyliques.

Selon un mode particulièrement préféré de réalisation de la présente invention, ledit groupement hydrocarboné à caractère anionique comprend le résultat de l'(homo)polymérisation radicalaire d'au moins un monomère anionique à insaturation éthylénique.

Selon un autre mode particulièrement préféré de réalisation de la présente invention, le dérivé siliconé mis en oeuvre comprend le résultat de la copolymérisation radicalaire entre d'une part au moins un monomère anionique présentant une insaturation éthylénique et d'autre part une silicone présentant dans sa chaine au moins un, et de préférence plusieurs, groupements fonctionels capables de venir réagir sur ladite insaturation éthylénique dudit monomère anionique en formant une liaison covalente, en particulier des groupements thio-fonctionnels.

Selon la présente invention, lesdits monomères anioniques à insaturation éthylénique sont de préférence choisis, seuls ou en mélanges, parmi les acides carboxyliques insaturés, linéaires ou ramifiés, éventuellement partiellement ou totalement neutralisés sous la forme d'un sel, ce ou ces acides carboxyliques insaturés pouvant être plus particulierement l'acide acrylique, l'acide méthacrylique, l'acide maléique, l'anhydride maléique, l'acide itaconique, l'acide fumarique et l'acide crotonique. Les sels convenables sont notamment les sels d'alcalins, d'alcalino-terreux et d'ammonium. On notera que, de même, dans le dérivé siliconé final, le groupement hydrocarboné à caractère anionique qui comprend le résultat de l'(homo)polymérisation radicalaire d'au moins un monomère anionique de type acide carboxylique insaturé peut être, après réaction, post-neutralisé avec une base (soude, ammoniaque,...) pour l'amener sous la forme d'un sel.

Des exemples de dérivés siliconés convenant à la mise en oeuvre de la présente invention, ainsi que leur mode particulier de préparation, sont notamment décrits dans les demandes de brevets EP-A- 0 582 152 et WO 93/23009, dont les enseignements sont totalement inclus dans la présente description à titre de références non limitatives. Parmi les dérivés siliconés décrits dans ces deux documents, il convient de ne retenir, comme indiqué précédemment, que ceux pour lesquels la chaine polysiloxane comporte des greffons polymériques anioniques capables de conférer au produit final son caractère hydrosoluble ou hydrodispersible.

Une famille de dérivés siliconés convenant particulièrement bien à la mise en oeuvre de la présente invention est constituée par les dérivés siliconés comportant dans leur structure le motif suivant : 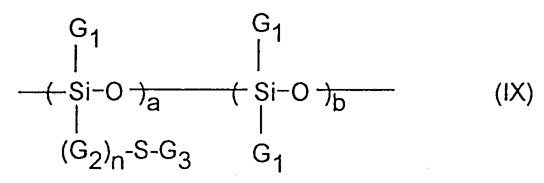 dans lequel G₁ représente l'hydrogène ou un radical alkyle en C₁-C₁₀ ou encore un radical phényle ; G₂ représente un groupe alkylène en C₁-C₁₀ ; G₃ représente un reste polymérique anionique résultant de l'(homo)polymérisation d'au moins un monomère anionique à insaturation éthylénique ; n est égal à 0 ou 1 ; a est un nombre entier pouvant être compris entre 1 et 50 ; et b est un nombre entier pouvant être compris entre 10 et 350.

De préférence, le motif de formule (IX) ci-dessus présente au moins l'une, et encore plus préférentiellement l'ensemble, des caractéristiques suivantes :
- G₁ est un radical alkyle, de préférence le radical méthyle,
- n est non nul, et G₂ représente un radical divalent en C₁-C₃, de préférence un radical propylène,
- G₃ représente un radical polymérique résultant de l'(homo)polymérisation d'au moins un monomère du type acide carboxylique insaturé, de préférence l'acide acrylique et/ou méthacrylique.

Le taux de groupement carboxylate dans le polymère final est de préférence compris entre 1 mole de carboxylate pour 200 g de polymère et 1 mole de carboxylate pour 5000 g de polymère.

De préférence, la masse moléculaire en nombre du polymère siliconé est comprise entre 10 000 et 1 000 000 environ, et encore plus préférentiellement entre 10 000 et 100 000 environ.
Des exemples de dérivés siliconés convenant particulièrement bien à la réalisation de la présente invention sont notamment ceux vendus par la Société 3M sous la dénomination commerciale de 〈〈SILICONE 〈〈PLUS〉〉 POLYMERS VS 80〉〉. Ces produits correspondent à des polydiméthylsiloxanes (PDMS) sur lesquels sont greffés, par l'intermédiaire d'un chainon de raccordement de type thiopropylène, des motifs polymères mixtes du type acide poly(méth)acrylique et du type ester poly(méth)acrylate d'isobutyle. Ces produits peuvent être classiquement obtenus par copolymérisation radicalaire entre d'une part une silicone de type polydiméthylsiloxane préalablement fonctionalisée par des groupements thiopropyl et, d'autre part, un mélange de monomères constitué d'acide (méth)acrylique et de (méth)acrylate d'isobutyle.

Les compositions capillaires conformes à l'invention renferment les dérivés siliconés définis ci-dessus à des teneurs pondérales qui peuvent être comprises entre 0,05 % et 10%, de préférence entre 0,1 % et 5% et encore plus préférentiellement entre 0,2 % et 3%, par rapport au poids total de la composition.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 3 et 10. De préférence, ce pH est compris entre 5,5 et 8. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents nacrants, des agents hydratants, des agents anti-pélliculaires ou anti-séborrhéiques, des vitamines, des filtres solaires et autres.

Bien entendu, l'homme de l'art veillera à choisir ce ou ces éventuels composés complémentaires et/ou leurs quantités de manière telle que les propriétés avantageuses attachées intrinsèquement à l'association ternaire (base lavante + polymère cationique + dérivé siliconé spécifique) conforme à l'invention ne soient pas, ou substantiellement pas, altérées par la ou les adjonctions envisagées.

Ces compositions peuvent se présenter sous la forme de liquides plus ou moins épaissis, de crêmes ou de gel et elles conviennent principalement au lavage, au soin et/ou le coiffage des cheveux. Elles peuvent aussi se présenter sous la forme de lotions à rincer.

Lorsque les compositions conformes à l'invention sont mises en oeuvre comme des shampooings classiques, elles sont simplement appliquées sur cheveux mouillés et la mousse générée par massage ou friction avec les mains est ensuite éliminée, après un éventuel temps de pause, par rinçage à l'eau, l'opération pouvant être répétée une ou plusieurs fois.

Comme indiqué précédemment, les compositions conformes à l'invention confèrent aux cheveux, après rinçage, un remarquable effet coiffant qui se manifeste notamment par une facilité de coiffage et de maintien, ainsi qu'un apport de volume et de légèreté, nettement améliorés.

Des exemples concrets, mais nullement limitatifs, illustrant l'invention vont maintenant être donnés.

### EXEMPLE 1

On a réalisé deux compositions de shampooing, l'une conforme à l'invention (composition A) et l'autre comparative (composition B), différant l'une de l'autre simplement par la nature du dérivé siliconé employé :

| | Composition A | Composition B |
|---|---|---|
| - Lauryléthersulphate de sodium (C12/C14 à 70/30) à 2,2 moles d'oxyde d'éthylène (MA = matière active) | 11,8 g MA | 11,8 g MA |
| - Cocoyl bétaine | 2,56 g MA | 2,56 g MA |
| - Gomme de xanthane | 1 g | 1 g |
| - Polymère cationique (*) | 0,5 g MA | 0,5 g MA |
| - Dérivé siliconé 1 (**) | 0,5 g MA | - |
| - Dérivé siliconé 2 (***) | - | 0,5 g MA |
| - Eau déminéralisée | qsp 100 g | qsp 100 g |

| | | |
|---|---|---|
| (*) : Copolymère chlorure de diméthyldiallylammonium/acrylamide (50/50), vendu en solution aqueuse sous la dénomination de MERQUAT 550 par la Société CALGON. | | |
| (**) : Polydiméthyl/méthylsiloxane à groupements propyl thio 3 acide polyméthacrylique/polyméthacrylate d'isobutyle, préneutralisé à l'ammoniaque et vendu en solution aqueuse sous la dénomination de VS 80 par la Société 3M. | | |
| (***) : Polydiméthyl/méthylsiloxane à groupements propyl thio 3 polyméthacrylate d'isobutyle, vendu dans une solution de silicone D4 sous la dénomination de VS 70 par la Société 3M. | | |

Des mèches de 2,7 g de cheveux décolorés (15,7 % de solubilité alcaline) d'une longueur de 24 cm sont préalablement humidifiées puis mises en contact avec 1 g de la composition A selon l'invention pendant 10 minutes puis rincées à l'eau. Les mèches encore humides sont ensuite enroulées sur des bigoudis de 2 cm de diamètre et de 7 cm de long. Les mèches sont ensuite séchées 30 minutes au casque-chauffant puis délicatement otées des bigoudis. On obtient ainsi des mèches sèches bouclées.

On procède selon le même mode opératoire que ci-dessus avec la composition comparative B.

On mesure alors la longueur (L) des mèches bouclées suspendues par leur seul poids devant un panneau gradué.

On les laisse ensuite se relaxer, toujours à l'état suspendu, pendant 4 heures à température ambiante.

La longueur des méches suspendues s'accroit alors d'une certaine longueur (ΔL).

Plus cet allongement ΔL est faible, meilleure est la tenue du coiffage dans le temps.

Ainsi, avec la composition B, l'allongement était de 3,6 cm, alors qu'il n'était que de 3 cm pour la composition A, soit une amélioration dans la réduction de l'allongement de plus de 16 %, ce qui traduit bien la meilleure tenue de coiffage obtenue grace au shampooing selon l'invention.

### EXEMPLE 2

On a réalisé deux compositions de shampooing, l'une conforme à l'invention (composition C) et l'autre comparative (composition D de l'art antérieur), différant l'une de l'autre simplement par la présence ou non d'un dérivé siliconé conforme à l'invention :

| | Composition C | Composition D |
|---|---|---|
| - Lauryléthersulphate de sodium (C₁₂/C₁₄ à 70/30) à 2,2 moles d'oxyde d'éthylène (MA = matière active) | 11,8 g MA | 11,8 g MA |
| - Cocoyl bétaine | 2,56 g MA | 2,56 g MA |
| - Polymère cationique (*) | 0,5 g MA | 2,5 g MA |
| - Dérivé siliconé 1 (**) | 2 g MA | - |
| - Eau déminéralisée | qsp 100 g | qsp 100 g |

| | | |
|---|---|---|
| (*) : identique à celui de l'exemple 1 (MERQUAT 550). | | |
| (**) : identique à celui de l'exemple 1 (VS 80). | | |

Des mèches de 2,7 g de cheveux décolorés (15,7 % de solubilité alcaline) d'une longueur de 24 cm sont préalablement humidifiées puis mises en contact une première fois avec 1 g de la composition C selon l'invention pendant 10 minutes puis rincées à l'eau, puis mises en contact une deuxième fois avec 1 g de cette même composition C à nouveau pendant 10 minutes, et enfin rincées une deuxième fois. Les mèches encore humides sont ensuite enroulées sur des bigoudis de 2 cm de diamètre et de 7 cm de long. Les mèches sont ensuite séchées 30 minutes au casque-chauffant puis délicatement otées des bigoudis. On obtient ainsi des mèches sèches bouclées.

On procède selon le même mode opératoire que ci-dessus avec la composition comparative D de l'art antérieur.

Un panel de 10 experts compare alors l'aptitude des mèches à la compression. Plus la déformation manuelle est aisée, moins l'effet de maintien de la coiffure est satisfaisant.

Pour les dix experts, la mèche traitée par la composition C selon l'invention est moins apte à la déformation que celle traitée par la composition comparative D, ce qui traduit bien la supériorité de l'effet coiffant attaché au shampooing selon l'invention.

## Revendications

1. Compositions capillaires détergentes, du type comprenant, dans un milieu cosmétiquement acceptable, une base lavante et au moins un polymère cationique, caractérisées par le fait qu'elles contiennent en outre au moins un dérivé siliconé hydrosoluble ou hydrodispersible comprenant une chaine principale siliconée sur laquelle est greffé au moins un groupement hydrocarboné à caractère anionique.

2. Compositions selon la revendication 1, caractérisées par le fait que ladite base lavante comprend un ou plusieurs tensioactifs choisis parmi des tensioactifs anioniques, amphotères, non ioniques, zwittérioniques, cationiques et leurs mélanges.

3. Compositions selon la revendication 1 ou 2, caractérisées par le fait que ladite base lavante est présente à une teneur pondérale comprise entre 4 % et 30 % par rapport au poids total de la composition.

4. Compositions selon la revendication 3, caractérisées par le fait que ladite teneur est comprise entre 10 % et 25 %.

5. Compositions selon la revendication 4, caractérisées par le fait que ladite teneur est comprise entre 12 % à 20 %.

6. Compositions selon l'une quelconque des revendications précédentes, caractérisées par le fait que ledit polymère cationique est choisi parmi les dérivés d'éther de cellulose quaternaires, les cyclopolymères, les polysaccharides cationiques, les polymères cationiques siliconés, et leurs mélanges.

7. Compositions selon la revendication 6, caractérisées par le fait que ledit cyclopolymère est choisis parmi les copolymères du chlorure de diméthyldiallylammonium et d'acrylamide ayant un poids moléculaire supérieur à 500 000.

8. Compositions selon l'une quelconque des revendications précédentes, caractérisées par le fait que ledit polymère cationique est présent à une teneur pondérale comprise entre 0,01 % et 10 % par rapport au poids total de la composition.

9. Composition selon la revendication 8, caractérisées par le fait que ladite teneur est comprise entre 0,05 % et 5 %.

10. Compositions selon la revendication 9, caractérisées par le fait que ladite teneur est comprise entre 0,1 % et 3 %.

11. Compositions selon l'une quelconque des revendications précédentes, caractérisées par le fait que ledit groupement hydrocarboné à caractère anionique comprend le résultat de l'(homo)polymérisation radicalaire d'au moins un monomère anionique à insaturation éthylénique.

12. Compositions selon l'une quelconque des revendications précédentes, caractérisées par le fait que ledit dérivé siliconé comprend le résultat de la copolymérisation radicalaire entre (i) au moins un monomère anionique à insaturation éthylénique et (ii) une silicone présentant dans sa chaine au moins un, et de préférence plusieurs, groupements fonctionels capables de venir réagir sur ladite insaturation éthylénique dudit monomère anionique en formant une liaison covalente.

13. Compositions selon la revendication 12, caractérisées par le fait que ledit groupement fonctionnel porté par ladite silicone est un groupement thio-fonctionnel.

14. Compositions selon l'une quelconque des revendications 11, 12 ou 13, caractérisées par le fait que ledit monomère anionique à insaturation éthylénique est choisi parmi les acides carboxyliques insaturés, linéaires ou ramifiés, éventuellement partiellement ou totalement neutralisés sous la forme d'un sel.

15. Compositions selon la revendication 14, caractérisées par le fait que lesdits acides carboxyliques sont choisis parmi l'acide acrylique, l'acide méthacrylique, l'acide maléique, l'anhydride maléique, l'acide itaconique, l'acide fumarique, l'acide crotonique et leurs mélanges

16. Compositions selon l'une quelconque des revendications précédentes, caractérisées par le fait que ledit dérivé siliconé est un dérivé siliconé comportant dans sa structure le motif suivant : 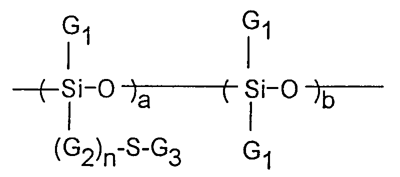 dans lequel G₁ représente l'hydrogène ou un radical alkyle en C₁-C₁₀ ou encore un radical phényle ; G₂ représente un groupe alkylène en C₁-C₁₀ ; G₃ représente un reste polymérique anionique résultant de l'(homo)polymérisation d'au moins un monomère anionique à insaturation éthylénique ; n est égal à 0 ou 1 ; a est un nombre entier compris entre 1 et 50; et b est un nombre entier compris entre 10 et 350.

17. Compositions selon la revendication 17, caractérisées par le fait que G₁ est un radical alkyle.

18. Compositions selon la revndication 17, caractérisées par le fait que ledit radical alkyle est le radical méthyle.

19. Compositions selon l'une quelconque des revendications 16 à 18, caractérisées par le fait que n est non nul, et que G₂ représente un radical divalent en C₁-C₃.

20. Compositions selon la revendication 19, caractérisées par le fait que ledit radical divalent est le radical propylène.

21. Compositions selon l'une quelconque des revendications 16 à 20, caractérisées par le fait que G₃ représente un radical polymérique résultant de l'(homo)polymérisation d'au moins un monomère du type acide carboxylique insaturé.

22. Compositions selon la revendication 21, caractérisées par le fait que ledit acide carboxylique insaturé est choisi parmi l'acide acrylique et/ou méthacrylique.

23. Compositions selon l'une des revendications 21 ou 22, caractérisées par le fait que le taux de groupement carboxylate dans le dérivé siliconé est compris entre 1 mole de carboxylate pour 200 g de dérivé siliconé et 1 mole de carboxylate pour 5000 g de dérivé siliconé.

24. Compositions selon l'une quelconque des revendications précédentes, caractérisées par le fait que ledit dérivé siliconé présente une masse moléculaire en nombre comprise entre 10 000 et 1 000 000 environ.

25. Compositions selon la revendication 24, caractérisées par le fait que ladite masse moléculaire est comprise entre 10 000 et 100 000 environ.

26. Compositions selon l'une quelconque des revendications précédentes, caractérisées par le fait que ledit dérivé siliconé est un polydiméthyl/méthylsiloxane à groupements propyl thio 3 acide polyméthacrylique/polyméthacrylate d'isobutyle.

27. Compositions selon l'une quelconque des revendications précédentes, caractérisées par le fait que ledit dérivé siliconé est présent à une teneur pondérale comprise entre 0,05 % et 10 % par rapport au poids total de la composition.

28. Compositions selon la revendication 27, caractérisées par le fait que ladite teneure est comprise entre 0,1 % et 5 %.

29. Compositions selon la revendication 28, caractérisées par le fait que ladite teneur est comprise entre 0,2 % et 3%.

30. Compositions selon l'une quelconque des revendications précédentes, caractérisées par le fait qu'elles présentent un pH compris entre 3 et 10.

31. Compositions selon la revendication 30, caractérisées par le fait que ledit pH est compris entre 5,5 et 8.

32. Utilisation d'une composition telle définie à l'une quelconque des revendications précédentes pour le nettoyage et/ou le soin et/ou le conditionnement et/ou le coiffage des cheveux.

33. Utilisation d'un dérivé siliconé tel que défini à l'une quelconque des revendications précédentes pour améliorer l'effet coiffant d'une composition capillaire détergente contenant au moins un polymère cationique.

## Claims

1. Detergent hair compositions of the type including, in a cosmetically acceptable medium, a washing base and at least one cationic polymer, characterized in that they additionally contain at least one water-soluble or water-dispersible silicone derivative including a silicone main chain onto which at least one hydrocarbon group of anionic nature is grafted.

2. Compositions according to Claim 1, characterized in that the said washing base includes one or more surfactants chosen from anionic, amphoteric, nonionic, zwitterionic, cationic surfactants and mixtures thereof.

3. Compositions according to Claim 1 or 2, characterized in that the said washing base is present in a weight content of between 4 % and 30 % relative to the total weight of the composition.

4. Compositions according to Claim 3, characterized in that the said content is between 10 % and 25 %.

5. Compositions according to Claim 4, characterized in that the said content is between 12 % and 20 %.

6. Compositions according to any one of the preceding claims, characterized in that the said cationic polymer is chosen from quaternary cellulose ether derivatives, cyclopolymers, cationic polysaccharides, silicone cationic polymers and mixtures thereof.

7. Compositions according to Claim 6, characterized in that the said cyclopolymer is chosen from copolymers of dimethyldiallylammonium chloride and of acrylamide which have a molecular weight higher than 500 000.

8. Compositions according to any one of the preceding claims, characterized in that the said cationic polymer is present in a weight content of between 0.01 % and 10 % relative to the total weight of the composition.

9. Composition according to Claim 8, characterized in that the said content is between 0.05 % and 5 %.

10. Compositions according to Claim 9, characterized in that the said content is between 0.1 % and 3 %.

11. Compositions according to any one of the preceding claims, characterized in that the said hydrocarbon group of anionic nature includes the result of the radical (homo)polymerization of at least one anionic monomer containing ethylenic unsaturation.

12. Compositions according to any one of the preceding claims, characterized in that the said silicone derivative includes the result of the radical copolymerization between (i) at least one anionic monomer containing ethylenic unsaturation and (ii) a silicone exhibiting in its chain at least one, and preferably several, functional groups capable of reacting with the said ethylenic unsaturation of the said anionic monomer by forming a covalent bond.

13. Compositions according to Claim 12, characterized in that the said functional group carried by the said silicone is a thiofunctional group.

14. Compositions according to any one of Claims 11, 12 or 13, characterized in that the said anionic monomer containing ethylenic unsaturation is chosen from linear or branched, unsaturated carboxylic acids, optionally partially or completely neutralized in the form of a salt.

15. Compositions according to Claim 14, characterized in that the said carboxylic acids are chosen from acrylic acid, methacrylic acid, maleic acid, maleic anhydride, itaconic acid, fumaric acid, crotonic acid and mixtures thereof.

16. Compositions according to any one of the preceding claims, characterized in that the said silicone derivative is a silicone derivative comprising the following unit in its structure: 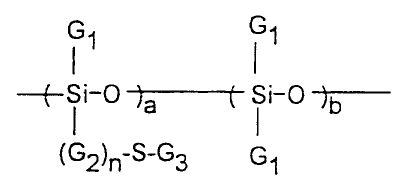 in which G₁ denotes hydrogen or a C₁-C₁₀ alkyl radical or else a phenyl radical, G₂ denotes a C₁-C₁₀ alkylene group, G₃ denotes an anionic polymeric residue resulting from the (homo)polymerization of at least one anionic monomer containing ethylenic unsaturation, n is equal to 0 or 1, a is an integer between 1 and 50, and b is an integer between 10 and 350.

17. Compositions according to Claim 17, characterized in that G₁ is an alkyl radical.

18. Compositions according to Claim 17, characterized in that the said alkyl radical is the methyl radical.

19. Compositions according to any one of Claims 16 to 18, characterized in that n is not zero and that G₂ denotes a C₁-C₃ divalent radical.

20. Compositions according to Claim 19, characterized in that the said divalent radical is the propylene radical.

21. Compositions according to any one of Claims 16 to 20, characterized in that G₃ denotes a polymeric radical resulting from the (homo)polymerization of at least one monomer of the unsaturated carboxylic acid type.

22. Compositions according to Claim 21, characterized in that the said unsaturated carboxylic acid is chosen from acrylic and/or methacrylic acid.

23. Compositions according to either of Claims 21 and 22, characterized in that the carboxylate group content in the silicone derivative is between 1 mole of carboxylate per 200 g of silicone derivative and 1 mole of carboxylate per 5000 g of silicone derivative.

24. Compositions according to any one of the preceding claims, characterized in that the said silicone derivative has a number molecular mass approximately between 10 000 and 1 000 000.

25. Compositions according to Claim 24, characterized in that the said molecular mass is approximately between 10 000 and 100 000.

26. Compositions according to any one of the preceding claims, characterized in that the said silicone derivative is a polydimethyl/methylsiloxane containing propyl thio 3 polymethacrylic acid/polyisobutyl methacrylate groups.

27. Compositions according to any one of the preceding claims, characterized in that the said silicone derivative is present in a weight content of between 0.05 % and 10 % relative to the total weight of the composition.

28. Compositions according to Claim 27, characterized in that the said content is between 0.1 % and 5 %.

29. Compositions according to Claim 28, characterized in that the said content is between 0.2 % and 3 %.

30. Compositions according to any one of the preceding claims, characterized in that they have a pH of between 3 and 10.

31. Compositions according to Claim 30, characterized in that the said pH is between 5.5 and 8.

32. Use of a composition as defined in any one of the preceding claims for the cleaning and/or care and/or conditioning and/or styling of hair.

33. Use of a silicone derivative as defined in any one of the preceding claims for improving the styling effect of a detergent hair composition containing at least one cationic polymer.

## Patentansprüche

1. Reinigende Zusammensetzungen für das Haar, die in einem kosmetisch akzeptablen Medium eine Waschgrundlage und mindestens ein kationisches Polymer enthalten,
**dadurch gekennzeichent, daß**
sie ferner mindestens ein wasserlösliches oder in Wasser dispergierbares Siliconderivat enthalten, das eine Siliconhauptkette aufweist, auf die mindestens eine Kohlenwasserstoffgruppe mit anionischer Eigenschaft gepfropft ist.

2. Zusammensetzungen nach Anspruch 1, dadurch gekennzeichnet, daß die Waschgrundlage einen oder mehrere grenzflächenaktive Stoffe enthält, die unter den anionischen, amphoteren, nicht ionischen, zwitterionischen, kationischen Tensiden und deren Gemischen ausgewählt ist.

3. Zusammensetzungen nach Anspruch 1 oder 2, dadurch gekennzeichnet, daß die Waschgrundlage in einem Gewichtsanteil im Bereich von 4 bis 30 %, bezogen auf das Gesamtgewicht der Zusammensetzung, vorliegt.

4. Zusammensetzungen nach Anspruch 3, dadurch gekennzeichnet, daß der Gehalt im Bereich von 10 bis 25 % liegt.

5. Zusammensetzungen nach Anspruch 4, dadurch gekennzeichnet, daß der Gehalt im Bereich von 12 bis 20 % liegt.

6. Zusammensetzungen nach einem der vorhergehenden Ansprüche, dadurch gekennzeichnet, daß das kationische Polymer unter den quaternären Celluloseetherderivaten, Cyclopolymeren, kationischen Polysacchariden, kationischen Siliconpolymeren und deren Gemischen ausgewählt ist.

7. Zusammensetzungen nach Anspruch 6, dadurch gekennzeichnet, daß das Cyclopolymer unter den Copolymeren von Dimethyldiallylammoniumchlorid und Acrylamid mit einem Molekülgewicht über 500000 ausgewählt ist.

8. Zusammensetzungen nach einem der vorhergehenden Ansprüche, dadurch gekennzeichnet, daß das kationische Polymer in einem Gewichtsanteil im Bereich von 0,01 bis 10 %, bezogen auf das Gesamtgewicht der Zusammensetzung, vorliegt.

9. Zusammensetzungen nach Anspruch 8, dadurch gekennzeichnet, daß der Gehalt im Bereich von 0,05 bis 5 % liegt.

10. Zusammensetzungen nach Anspruch 9, dadurch gekennzeichnet, daß der Gehalt im Bereich von 0,1 bis 3 % liegt.

11. Zusammensetzungen nach einem der vorhergehenden Ansprüche, dadurch gekennzeichnet, daß die Kohlenwasserstoffgruppe mit anionischer Eigenschaft das Ergebnis der radikalischen (Homo)polymerisation mindestens eines anionischen Monomers mit ethylenisch ungesättigter Bindung umfaßt.

12. Zusammensetzungen nach einem der vorhergehenden Ansprüche, dadurch gekennzeichnet, daß das Siliconderivat das Ergebnis der radikalischen Copolymerisation (i) mindestens eines anionischen Monomers mit ethylenisch ungesättigter Bindung und (ii) eines Silicons, das in seiner Kette mindestens eine und vorzugsweise mehrere funktionelle Gruppen aufweist, die mit der ethylenisch ungesättigten Bindung des anionischen Monomers unter Bildung einer kovalenten Bindung reagieren können.

13. Zusammensetzungen nach Anspruch 12, dadurch gekennzeichnet, daß die von dem Silicon getragene funktionelle Gruppe eine thiofunktionelle Gruppe ist.

14. Zusammensetzungen nach einem der Ansprüche 11, 12 oder 13, dadurch gekennzeichnet, daß das anionische Monomer mit ethylenisch ungesättigter Bindung unter den ungesättigten, geradkettigen oder verzweigten, gegebenenfalls teilweise oder vollständig in Form eines Salzes neutralisierten Carbonsäuren ausgewählt ist.

15. Zusammensetzungen nach Anspruch 14, dadurch gekennzeichnet, daß die Carbonsäuren unter Acrylsäure, Methacrylsäure, Maleinsäure, Maleinsäureanhydrid, Itaconsäure, Fumarsäure, Crotonsäure und deren Gemischen ausgewählt ist.

16. Zusammensetzungen nach einem der vorhergehenden Ansprüche, dadurch gekennzeichnet, daß das Siliconderivat ein Siliconderivat ist, das in seiner Struktur die folgende Einheit enthält: 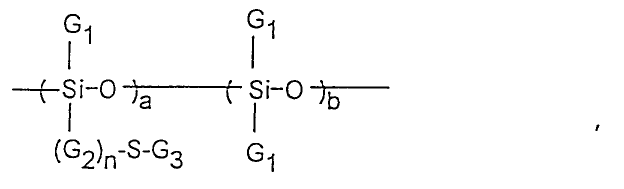 worin G₁ Wasserstoff oder C₁₋₁₀-Alkyl oder auch Phenyl bedeutet; G₂ bedeutet C₁₋₁₀-Alkylen; G₃ bedeutet eine anionische Polymergruppe, die aus der (Homo)polymerisation mindestens eines anionischen Polymers mit ethylenisch ungesättigter Bindung stammt; n ist Null oder 1, a ist eine ganze Zahl im Bereich von 1 bis 50; und b ist eine ganze Zahl im Bereich von 10 bis 350.

17. Zusammensetzungen nach Anspruch 16, dadurch gekennzeichnet, daß G₁ eine Alkylgruppe ist.

18. Zusammensetzungen nach Anspruch 17, dadurch gekennzeichnet, daß die Alkylgruppe Methyl ist.

19. Zusammensetzungen nach einem der Ansprüche 16 bis 18, dadurch gekennzeichnet, daß n nicht Null ist und G₂ eine zweiwertige Gruppe mit 1 bis 3 Kohlenstoffatomen bedeutet.

20. Zusammensetzungen nach Anspruch 19, dadurch gekennzeichnet, daß die zweiwertige Gruppe die Propylengruppe ist.

21. Zusammensetzungen nach einem der Ansprüche 16 bis 20, dadurch gekennzeichnet, daß G₃ eine Polymergruppe bedeutet, die aus der (Homo)polymerisation mindestens eines Monomers vom Typ ungesättigte Carbonsäure stammt.

22. Zusammensetzungen nach Anspruch 21, dadurch gekennzeichnet, daß die ungesättigte Carbonsäure unter Acrylsäure und/oder Methacrylsäure ausgewählt ist.

23. Zusammensetzungen nach einem der Ansprüche 21 oder 22, dadurch gekennzeichnet, daß der Gehalt an Carboxylatgruppen in dem Siliconderivat im Bereich von 1 mol Carboxylat pro 200 g Siliconderivat und 1 mol Carboxylat pro 5000 g Siliconderivat liegt.

24. Zusammensetzungen nach einem der vorhergehenden Ansprüche, dadurch gekennzeichnet, daß das Siliconderivat eine zahlenbezogene Molekülmasse im Bereich von etwa 10000 bis 1000000 aufweist.

25. Zusammensetzungen nach Anspruch 24, dadurch gekennzeichnet, daß die Molekülmasse im Bereich von etwa 10000 bis 100000 liegt.

26. Zusammensetzungen nach einem der vorhergehenden Ansprüche, dadurch gekennzeichnet, daß das Siliconderivat ein Polydimethyl/methylsiloxan mit 3-Propylthiopolymethacrylsäure/Polymethacrylsäureisobutylestergruppen ist.

27. Zusammensetzungen nach einem der vorhergehenden Ansprüche, dadurch gekennzeichnet, daß das Siliconderivat in einem Gewichtsanteil im Bereich von 0,05 bis 10 %, bezogen auf das Gesamtgewicht der Zusammensetzung, vorliegt.

28. Zusammensetzungen nach Anspruch 27, dadurch gekennzeichnet, daß der Anteil im Bereich von 0,1 bis 5 % liegt.

29. Zusammensetzungen nach Anspruch 28, dadurch gekennzeichnet, daß der Gehalt im Bereich von 0,2 bis 3 % liegt.

30. Zusammensetzungen nach einem der vorhergehenden Ansprüche, dadurch gekennzeichnet, daß sie einen pH-Wert im Bereich von 3 bis 10 aufweisen.

31. Zusammensetzungen nach Anspruch 30, dadurch gekennzeichnet, daß der pH-Wert im Bereich von 5,5 bis 8 liegt.

32. Verwendung einer Zusammensetzung nach einem der vorhergehenden Ansprüche zur Reinigung und/oder Pflege und/oder Konditionierung und/oder zum Frisieren der Haare.

33. Verwendung eines Siliconderivats nach einem der vorhergehenden Ansprüche zur Verbesserung der Frisierwirkung einer Reinigungszusammensetzung für das Haar, die mindestens ein kationisches Polymer enthält.
